# FASCICULE DE BREVET EUROPEEN

(11) **EP 1 337 147 B1**
(45) Date de publication et mention de la délivrance du brevet: **13.12.2006**
(21) Numéro de dépôt: 01980591.0
(22) Date de dépôt: 10.10.2001
(51) Int. Cl.: A01N 25/34

(54) **BILLE ET BATONNET VISCOELASTIQUES,NOTAMMENT POUR LA PREVENTION OU LE SOIN DES EFFETS DE L'HYPERHYDROSE PLANTAIRE**
VISKOELASTISCHE KUGEL UND STAB, ZUR VORBEUGUNG UND BEHANDLUNG VON EFFEKTEN DER FUSSSOHLEN-HYPERHYDROSE
VISCOELASTIC BALL AND STICK, DESTINED FOR PREVENTING OR TREATING EFFECTS OF PLANTAR HYPERHYDROSIS

(30) Priorité: 11.10.2000 FR 0012979
(43) Date de publication de la demande: 27.08.2003
(73) Titulaire: Millet Innovation, 26270 Loriol sur Drome (FR)
(72) Inventeur: MILLET, Jean-Claude, F-26800 Etoile sur Rhône (FR); LOPEZ, Isabelle, F-26000 Valence (FR)
(74) Mandataire: Marchand, André
(86) Numéro de dépôt international: PCT/FR2001/003112
(87) Numéro de publication internationale: WO 2002/030197

(56) Documents cités:
- EP-A- 0 272 918
- WO-A-92/10933
- FR-A- 2 372 623
- US-A- 6 015 570

## Description

La présente invention concerne l'application d'une substance active à une zone du corps humain.

La présente invention concerne également la prévention ou le soin des effets de l'hyperhydrose plantaire.

La présente invention concerne notamment un procédé pour supprimer, sinon limiter, l'apparition de mauvaises odeurs au niveau des pieds.

La sudation est un processus physiologique normal et indispensable pour l'hydratation de la peau et l'élimination des toxines. Cependant, il arrive que les glandes sudoripares s'emballent et produisent une sudation excessive appelée *hyperhydrose.* Au niveau des pieds, cet excès de sudation s'accompagne souvent d'un phénomène de macération qui crée un milieu favorable à la prolifération de bactéries ou de champignons. Les bactéries dégradent les composés azotés de la sueur qui devient malodorante - on parle alors de *bromhydrose -* tandis que les champignons se localisent dans les zones les moins ventilées, en particulier dans les troisième et quatrième espaces interdigitaux, en provoquant des démangeaisons et rougeurs ou *mycose.* Ainsi, les deux conséquences de l'hyperhydrose, à savoir la *bromhydrose* et la *mycose,* s'avèrent encore plus gênantes pour la personne qui transpire que la sueur elle-même.

Pour prévenir ces pathologies, on trouve sur le marché des antisudoriques et des désodorisants. L'antisudorique agit sur la béance des pores sudoraux pour limiter la sudation. Il se présente sous des formes galéniques variées (lotion, crème, bâton applicateur, aérosol) mais se révèle irritant dans le cas d'une utilisation prolongée. Le désodorisant limite l'apparition de l'odeur nauséabonde tout en respectant le processus physiologique. Outre les produits classiques tels que la poudre à mettre dans les chaussures ou les "semelles hygiéniques", citons la mise au point récente d'une fibre polyamide bactériostatique qui pourrait être utilisée pour la confection de chaussettes afin de limiter la formation de mauvaises odeurs.

Ainsi, en cas d'infection ou de mycose sur les pieds, la solution proposée à ce jour consiste en l'application d'une crème ou d'une poudre (médicament) sur les zones lésées. Toutefois, aucun des produits actuellement sur le marché ne représente une solution satisfaisante qui soit optimisée à la fois du point de vue de l'efficacité, de la fonctionnalité et de la non-toxicité, pour agir aussi bien en prévention qu'en cure. En effet, comme on l'a indiqué plus haut, les antisudoriques sont irritants dans le cas d'une utilisation prolongée. Or, comme leur efficacité est de courte durée, ce type de produit doit être appliqué en continu. L'apparition d'effets secondaires est ainsi inévitable.

L'utilisation d'un désodorisant est moins traumatisante pour le corps mais s'avère peu pratique et n'est généralement pas appréciée des utilisateurs, que l'application du désodorisant se fasse par aspersion ou par dépôt d'une poudre.

D'autre part, l'utilisation d'une semelle dite hygiénique, ayant subi un traitement antibactérien, peut être une bonne solution à des problèmes de transpiration mais nécessite des chaussures assez grandes pour que le pied conserve son aisance en présence de la semelle. Dans le cas contraire, on peut voir apparaître des traumatismes d'ordre mécanique comme des durillons.

De plus, les semelles et autres chaussettes antibactériennes couvrent des zones de moyenne transpiration, au niveau du talon par exemple, et agissent peu dans des régions critiques, telles que les troisième et quatrième espaces interdigitaux.

Un objectif de la présente invention est de prévoir un procédé pour supprimer, sinon limiter, l'apparition de mauvaises odeurs au niveau du pied.

Un autre objectif de la présente invention est de prévoir un procédé permettant d'appliquer une substance déterminée à une zone du corps humain, notamment une substance active visant à supprimer ou limiter l'apparition de mauvaises odeurs au niveau du pied.

Encore un autre objectif de la présente invention est de prévoir un produit pour la prévention ou le soin des conséquences de l'hyperhydrose plantaire, qui soit applicable au plus près d'une zone critique du pied, qui soit pratique à l'emploi, qui offre une efficacité satisfaisante à long terme et qui puisse être utilisé de façon continue sans effets secondaires.

Pour atteindre ces objectifs, la présente invention prévoit un procédé pour appliquer une substance active dans la région du pied, mis en oeuvre pour des raisons esthétiques non thérapeutiques, comprenant l'application dans au moins un espace interdigital d'un matériau viscoélastique dont la forme est prévue pour être insérée dans l'espace interdigital visé et y être maintenue par coincement entre des orteils, le matériau contenant une substance active se libérant lentement au contact de la peau.

Selon un mode de réalisation, le matériau viscoélastique est un gel silicone.

De préférence, le gel silicone présente des propriétés mécaniques proches de celles du capiton plantaire humain.

Selon un mode de réalisation, la substance est choisie dans le groupe comprenant les fongistatiques, les fongicides, les bactériostatiques et les bactéricides, pour supprimer sinon limiter l'apparition de mauvaises odeurs au niveau du pied.

Selon un mode de réalisation, le matériau viscoélastique est de forme sphérique ou ovoïde et d'un diamètre adapté à l'espace interdigital visé.

Selon un mode de réalisation, le matériau viscoélastique est de forme cylindrique et d'un diamètre adapté à l'espace interdigital visé.

Selon un mode de réalisation, le matériau viscoélastique présente un diamètre minimal de l'ordre de 4 mm.

La présente invention concerne également un produit pour l'application d'une substance active sur une zone du pied, comprenant un matériau viscoélastique de forme prévue pour être insérée dans un espace interdigital et y être maintenue par coincement entre des orteils, et au moins une substance active incorporée dans le matériau viscoélastique, se libérant lentement du matériau viscoélastique lorsque celui-ci est agencé au contact de la peau.

Selon un mode de réalisation, le matériau viscoélastique est un gel silicone.

De préférence, le gel silicone présente des propriétés mécaniques proches de celles du capiton plantaire humain.

Selon un mode de réalisation visant la prévention ou le soin des conséquences de l'hyperhydrose plantaire, dans lequel la substance active est choisie dans le groupe comprenant les fongistatiques, les fongicides, les bactériostatiques et les bactéricides.

Selon un mode de réalisation, le produit est de forme sphérique ou ovoïde et d'un diamètre adapté à l'espace interdigital visé.

Selon un mode de réalisation, le produit est de forme cylindrique et d'un diamètre adapté à l'espace interdigital visé.

De façon générale, la présente invention concerne également un produit pour l'application d'une substance active sur une zone du corps humain, comprenant un matériau viscoélastique de forme sphérique ou ovoïde présentant un diamètre minimal de l'ordre de 2 mm pour une application à l'oeil ou de l'ordre de 4 mm pour une application à une autre zone du corps humain, et au moins une substance active incorporée dans le matériau viscoélastique, se libérant lentement du matériau viscoélastique lorsque celui-ci est agencé au contact de la peau.

La présente,invention concerne également un produit pour l'application d'une substance active sur une zone du corps humain, comprenant un matériau viscoélastique de forme cylindrique et d'un diamètre minimal de 4 mm, et au moins une substance active incorporée dans le matériau viscoélastique, se libérant lentement du matériau viscoélastique lorsque celui-ci est agencé au contact de la peau.

Selon un mode de réalisation, le matériau viscoélastique est un gel silicone.

De préférence, le gel silicone présente des propriétés mécaniques proches de celles du tissu humain de la zone du corps humain visée.

La présente invention concerne également un procédé de fabrication d'un produit viscoélastique de forme sphérique ou ovoïde destiné à l'application d'une substance active sur une zone du corps humain, comprenant les étapes suivantes : préparation d'un mélange comprenant au moins un composé destiné à réticuler pour former après réticulation un matériau ayant des propriétés viscoélastiques, et au moins une substance active miscible avec le composé, introduction d'un volume' déterminé du mélange dans un récipient contenant un fluide non miscible avec le mélange, et réticulation du volume de mélange immergé dans le fluide.

Selon un mode de réalisation visant la fabrication d'un produit viscoélastique en gel silicone, le mélange comprend au moins une huile silicone,

Selon un mode de réalisation, la substance active est choisie dans le groupe comprenant les fongistatiques, les fongicides, les bactériostatiques et les bactéricides.

Selon un mode de réalisation, la masse volumique du fluide est choisie de manière que le mélange reste en suspension dans le fluide tout en étant immergé, ou descende en direction du fond du récipient avec une vitesse suffisamment lente pour assurer sa réticulation avant que le fond du récipient ne soit atteint.

Selon un mode de réalisation, le fluide est chauffé afin d'accélérer le processus de réticulation.

Ces objets, caractéristiques et avantages ainsi que d'autres de la présente invention seront exposés plus en détail dans la description suivante d'un produit selon l'invention, d'une utilisation de ce produit et d'un procédé de fabrication de ce produit, faite à titre non limitatif en relation avec les figures jointes parmi lesquelles :
- la figure 1 représente un pied humain et un mode de réalisation d'un produit selon l'invention,
- la figure 2 est une vue agrandie d'un espace interdigital apparaissant en figure 1.

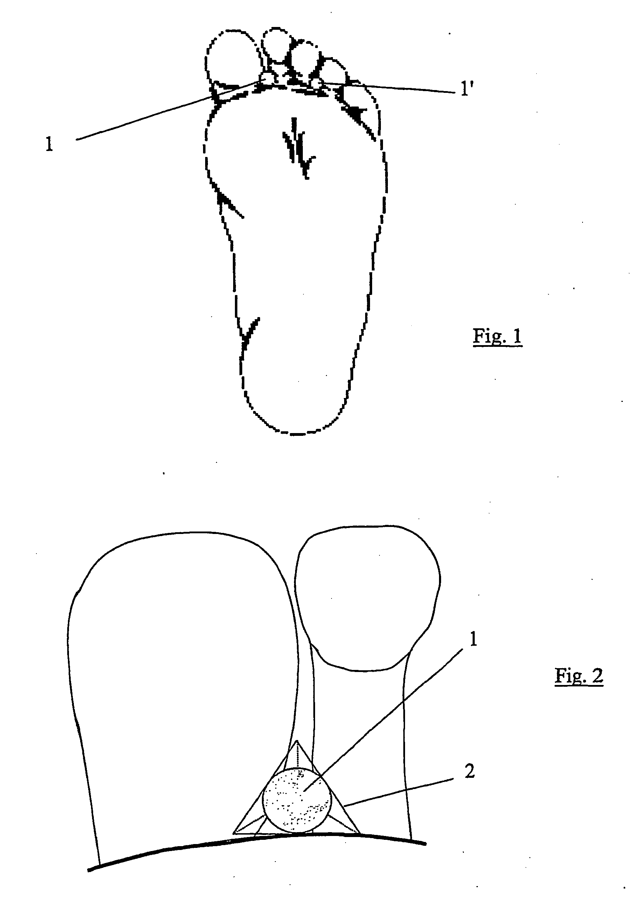

La figure 1 représente un mode de réalisation préféré d'un produit selon l'invention, destiné à la prévention de la bromhydrose (mauvaises odeurs) ou au soin des mycoses et autres infections.

Le produit selon l'invention se présente sous la forme d'une bille viscoélastique 1 d'un diamètre de quelques millimètres, de préférence de l'ordre de 6 à 10 mm, ayant été imprégnée d'une substance active d'une manière qui sera décrite plus loin. La bille 1 est agencée dans un espace interdigital devant être préservé ou traité, et libère lentement la substance active. Sur la figure 1, la bille 1 est agencée dans le quatrième espace interdigital et une deuxième bille 1' selon l'invention est représentée agencée dans le deuxième espace interdigital.

La bille 1 est réalisée en un matériau viscoélastique et est ainsi apte à occuper l'espace interdigital quelle que soit la morphologie du pied, en ne provoquant aucune gêne à l'utilisateur. Le matériau viscoélastique est de préférence un gel silicone lavable et réutilisable jusqu'à épuisement de la substance active, le lavage n'entraînant pas la dissolution de la substance active. Ce gel silicone est de préférence biocompatible et présente de préférence des propriétés mécaniques similaires au capiton plantaire humain afin de protéger contre le stress épidermique la zone du pied au contact de la bille. Parmi les gels silicone biocompatibles présentant de telles propriétés mécaniques, on connaît notamment les compositions de polymères silicone décrites dans le brevet FR-2 712 487, qui présentent l'avantage de reproduire les propriétés mécaniques du capiton plantaire naturel en compression et en torsion. Des gels silicone ayant de telles propriétés sont également commercialisés par la demanderesse sous les appellations EPITHELIUM 26, EPITHELIUM 27 ou EPITHELIUM 27+. Ils présentent des qualités de contact exceptionnelles et donnent des résultats remarquables en termes de confort, qui les rendent aptes à l'application prévue par la présente invention.

La figure 2 est une vue agrandie du creux formé par la quatrième espace interdigital. On voit que ce creux, qui se trouve entre l'avant du pied et deux orteils, est sensiblement en forme de pyramide 2, de sorte que la bille 1 selon l'invention peut s'y loger en entrant en contact avec la peau sur au moins trois points, ce qui permet de s'affranchir de tout autre moyen de fixation. De plus, le matériau viscoélastique formant la bille peut être choisi sensiblement adhésif, voire autoadhésif. Il s'agit par exemple d'un gel silicone du type EPITHELIUM 27+.

La substance active contenue par la bille 1 est du type fongistatique et/ou bactériostatique pour une application de l'invention à la prévention des odeurs désagréables, ou du type bactéricide et/ou fongicide pour une application de l'invention au traitement des infections et mycoses. Il s'agit par exemple du triclosan (fongistatique et bactériostatique) ou du nitrate d'éconazole (bactéricide et fongicide). Elle est libérée lentement (relarguée) et en continu par la bille, dans la zone d'application de la bille.

En pratique, la zone d'influence de la substance active est de l'ordre de 40 mm autour de la bille, de sorte qu'une seule bille permet de prévenir les odeurs ou de traiter des infections sur au moins deux orteils.

On décrira maintenant un mode de réalisation avantageux d'une bille en gel silicone selon l'invention, ne nécessitant pas de moule pour le maintien du composant de base pendant le processus de réticulation (transformation en gel). Le composant de base utilisé est une composition comprenant une ou plusieurs huiles silicone, par exemple la composition à trois huiles silicone décrite dans le brevet FR 2 712 487 précité. On incorpore dans la composition au moins une substance active, choisie en fonction de l'application visée (prévention ou traitement). Cette substance doit être miscible avec les huiles silicone. Il s'agit par exemple de triclosan, incorporé selon un dosage représentant 0,1 à 0,3% en poids du mélange initial.

Un volume dosé du mélange huiles silicone/substance active est déposé à l'aide d'une buse (tube, seringue...) dans un milieu fluide contenu dans un récipient. Le fluide n'est pas miscible avec le mélange selon l'invention et son contact avec le mélange n'inhibe pas la réaction de réticulation. La masse volumique du fluide est choisie de manière que le volume de mélange déposé dans le récipient se trouve en suspension dans le milieu ou descende très lentement en direction du fond du récipient durant la réticulation. Le volume de mélange ne doit pas remonter à la surface du fluide et doit rester immergé. Il ne doit pas non plus descendre trop vite car il risquerait de s'écraser au fond du récipient avant que le processus de réticulation ne soit terminé. Lorsque ces conditions sont respectées, comme la viscosité du fluide est moyenne et que les forces de tension superficielle sont prédominantes, le volume de mélange prend naturellement une forme sphérique afin de minimiser son énergie de surface, et se réticule en gardant une forme sphérique. On obtient ainsi un objet que l'on appelle, selon l'invention, une bille.

En pratique, le fluide est par exemple de l'eau dans laquelle on a incorporé des solutions contenant des sucres ou des sels afin d'obtenir la masse volumique souhaitée. I1 peut être chauffé pour accélérer le processus de réticulation, par exemple dans une gamme de températures allant de 40 à 60°C.

Dans le cas où la masse volumique du fluide ne peut être ajustée convenablement, tout autre moyen pour maintenir la goutte en suspension peut être envisagé, notamment l'utilisation d'un flux ascendant ou descendant pour compenser les forces exercées sur la goutte.

A l'usage, il apparaît que le produit selon l'invention répond parfaitement aux objectifs fixés. Il est fonctionnel et non salissant. Il suffit de glisser la bille le matin dans l'espace interdigital à traiter ou à protéger et de l'enlever le soir. La sphère se déforme et occupe l'espace disponible sans gêne pour l'utilisateur et sans encombrement supplémentaire dans la chaussure. La bille agit efficacement et en continu dans la zone "critique" et seulement dans cette zone. L'action est ainsi parfaitement ciblée et ne modifie pas l'équilibre de la flore cutanée dans les zones "saines".

L'utilisation d'une bille selon l'invention n'entraîne aucun effet second indésirable, qu'il soit d'origine mécanique ou chimique. De plus, la bille est lavable et réutilisable jusqu'à épuisement de la substance active. Ainsi, on a constaté que la "durée de vie" d'une bille selon l'invention, c'est-à-dire le temps de relargage de la substance active, est supérieur à un mois avec lavage quotidien de la bille.

Diverses variantes de réalisation de la présente invention peuvent être envisagées tant en ce qui concerne la forme du produit qu'en ce qui concerne sa composition ou son application. Notamment, au lieu d'être placée dans un espace interdigital, la bille peut être placée directement sous l'orteil si une zone critique à soigner se situe à cet endroit. La forme recourbée des quatrième et cinquième orteils permet le maintien en place de la bille sans nécessiter un autre mode de fixation.

D'autre part, le produit selon l'invention peut revêtir diverses formes autres que celle d'une bille, par exemple une forme ovoïde ou une forme cylindrique (bâtonnet), et de façon générale toute forme adaptée à la morphologie plantaire ou à la morphologie de l'espace interdigital. De plus, l'espace interdigital au sens de la présente invention comprend également le creux formé par une rangée d'orteils, dans lequel un bâtonnet viscoélastique selon l'invention, chargé en matière active, peut être agencé transversalement.

Outre les gels silicone, des gels aqueux (hydrogels) tels les gels obtenus par réticulation de solutions polysaccharides, peuvent également être utilisés pour réaliser un produit selon l'invention.

Egalement, un produit selon l'invention peut être utilisé pour libérer lentement tout type de substance active, par exemple une substance odoriférante et cicatrisante comme de l'huile essentielle de lavande.

La présente invention est ainsi utilisable pour l'application lente et régulière d'une substance déterminée sur des zones du corps humain telles que le buste, les bras ou les jambes, voire le visage.

Ainsi, une application avantageuse de la présente invention consiste à prévoir une bille viscoélastique destinée à être appliquée dans un coin de l'oeil pour la libération lente d'une substance active thérapeutique.

De préférence, une bille ou un ovoïde selon l'invention présente un diamètre minimal de l'ordre de 2mm pour une application à l'oeil humain et un diamètre minimal de l'ordre de 6 mm pour une application à un espace interdigital du pied. De préférence toujours, un cylindre ou bâtonnet selon l'invention présente un diamètre minimal de l'ordre de 4 mm. De préférence toujours, une bille un ovoïde ou un bâtonnet selon l'invention est réalisé en un gel qui présente des propriétés mécaniques proches de celles du tissu humain de la zone du corps humain visée, afin d'être indolore pour l'utilisateur et de ne pas provoquer de sensation de dureté qui rendrait le produit inconfortable et pourrait en outre conduire à l'apparition d'hématomes.

De façon générale, la présente invention repose sur le fait qu'un gel viscoélastique présente la propriété de libérer lentement une substance mélangée à la composition de base avant réticulation de cette dernière. Une telle propriété peut s'expliquer par le fait que la substance active, qui est miscible dans la composition de base, ne réticule pas et se trouve sous forme libre à l'intérieur du réseau microscopique existant après réticulation de la composition de base, ce qui lui permet de migrer vers la périphérie du matériau.

## Revendications

1. Procédé pour appliquer une substance active dans la région du pied (2), mis en oeuvre pour des raisons esthétiques non thérapeutiques, **caractérisé en ce qu'**il comprend l'application dans au moins un espace interdigital (2) d'un matériau viscoélastique (1, 1') dont la forme est prévue pour être insérée dans l'espace interdigital visé et y être maintenue par coincement entre des orteils, le matériau contenant une substance active se libérant lentement au contact de la peau.

2. Procédé selon la revendication 1, dans lequel le matériau viscoélastique est un gel silicone.

3. Procédé selon la revendication 2, dans lequel le gel silicone présente des propriétés mécaniques proches de celles du capiton plantaire humain.

4. Procédé selon l'une des revendications 1 à 3, dans lequel la substance est une substance visant à supprimer sinon limiter l'apparition de mauvaises odeurs au niveau du pied.

5. Procédé selon l'une des revendications 1 à 4, dans lequel la matériau viscoélastique est de forme sphérique ou ovoïde et d'un diamètre adapté à l'espace interdigital visé.

6. Procédé selon l'une des revendications 1 à 4, dans lequel le matériau viscoélastique est de forme cylindrique et d'un diamètre adapté à l'espace interdigital visé.

7. Procédé selon l'une des revendications 5 et 6, dans lequel le matériau viscoélastique présente un diamètre minimal de l'ordre de 4 mm.

8. Produit (1, 1') pour l'application d'une substance active sur une zone du pied, **caractérisé en ce qu'**il comprend :
- un matériau viscoélastique de forme prévue pour être insérée dans un espace interdigital (2) et y être maintenue par coincement entre des orteils, et
- au moins une substance active incorporée dans le matériau viscoélastique, se libérant lentement du matériau viscoélastique lorsque celui-ci est agencé au contact de la peau.

9. Produit selon la revendication 8, dans lequel le matériau viscoélastique est un gel silicone.

10. Produit selon la revendication 9, dans lequel le gel silicone présente des propriétés mécaniques proches du capiton plantaire humain.

11. Produit selon l'une des revendications 8 à 10, pour la prévention ou le soin des conséquences de l'hyperhydrose plantaire, dans lequel la substance active est choisie dans le groupe comprenant les fongistatiques, les fongicides, les bactériostatiques et les bactéricides.

12. Produit selon l'une des revendications 8 à 11, de forme sphérique ou ovoïde et d'un diamètre adapté à l'espace interdigital visé.

13. Produit selon l'une des revendications 8 à 11, de forme cylindrique et d'un diamètre adapté à l'espace interdigital visé.

14. Produit (1, 1') pour l'application d'une substance active sur une zone du corps humain, **caractérisé en ce qu'**il comprend :
- un matériau viscoélastique de forme sphérique ou ovoïde, présentant un diamètre minimal de l'ordre de 2 mm pour une application à l'oeil ou de l'ordre de 4 mm pour une application à une autre zone du corps humain, et
- au moins une substance active incorporée dans le matériau viscoélastique, se libérant lentement du matériau viscoélastique lorsque celui-ci est agencé au contact de la peau.

15. Produit selon la revendication 14, dans lequel le matériau viscoélastique est un gel silicone.

16. Produit selon la revendication 15, dans lequel le gel silicone présente des propriétés mécaniques proches de celles du tissu humain de la zone du corps humain visée.

17. Produit pour l'application d'une substance active sur une zone du corps humain, **caractérisé en ce qu'**il comprend :
- un matériau viscoélastique de forme cylindrique et d'un diamètre minimal de 4 mm, et
- au moins une substance active incorporée dans le matériau viscoélastique, se libérant lentement du matériau viscoélastique lorsque celui-ci est agencé au contact de la peau.

18. Produit selon la revendication 17, dans lequel le matériau viscoélastique est un gel silicone.

19. Produit selon la revendication 18, dans lequel le gel silicone présente des propriétés mécaniques proches de celles du tissu humain de la zone du corps humain visée.

20. Procédé de fabrication d'un produit viscoélastique (1, 1') de forme sphérique ou ovoïde destiné à l'application d'une substance active sur une zone (2) du corps humain, **caractérisé en ce qu'**il comprend les étapes suivantes :
- préparation d'un mélange comprenant au moins un composé destiné à réticuler pour former après réticulation un matériau ayant des propriétés viscoélastiques, et au moins une substance active miscible avec le composé,
- introduction d'un volume déterminé du mélange dans un récipient contenant un fluide non miscible avec le mélange, et
- réticulation du volume de mélange immergé dans le fluide.

21. Procédé selon la revendication 20, pour la fabrication d'un produit viscoélastique en gel silicone, dans lequel le mélange comprend au moins une huile silicone.

22. Procédé selon l'une des revendications 20 et 21, dans lequel la substance active est choisie dans le groupe comprenant les fongistatiques, les fongicides, les bactériostatiques et les bactéricides.

23. Procédé selon l'une des revendications 20 à 22, dans lequel la masse volumique du fluide est choisie de manière que le mélange reste en suspension dans le fluide tout en étant immergé, ou descende en direction du fond du récipient avec une vitesse suffisamment lente pour assurer sa réticulation avant que le fond du récipient ne soit atteint.

24. Procédé selon l'une des revendications 20 à 23, dans lequel le fluide est chauffé afin d'accélérer le processus de réticulation.

## Claims

1. A method for applying an active substance in the area of the foot (2), implemented for non-therapeutic aesthetic reasons, **characterized in that** it comprises the application in at least one interdigital space (2) of a viscoelastic material (1, 1') the shape of which is designed for being inserted into the interdigital space targeted and for being held therein by wedging between toes, the material containing an active substance that is slowly released when in contact with the skin.

2. Method according to claim 1, wherein the viscoelastic material is a silicone gel.

3. Method according to claim 2, wherein the silicone gel has mechanical properties similar to that of the human footpad.

4. Method according to one of claims 1 to 3, wherein the substance is a substance aiming to remove or to limit the appearance of unpleasant odors at the foot.

5. Method according to one of claims 1 to 4, wherein the viscoelastic material is spherical or ovoid in shape and of a diameter adapted to the interdigital space targeted.

6. Method according to one of claims 1 to 4, wherein the viscoelastic material is cylindrical in shape and of a diameter adapted to the interdigital space targeted.

7. Method according to one of claims 5 and 6, wherein the viscoelastic material has a minimum diameter in the order of 4 mm.

8. A product (1, 1') for applying an active substance to an area of the foot, **characterized in that** it comprises:
- a viscoelastic material the shape of which is designed for being inserted into an interdigital space (2) and for being held therein by wedging between toes, and
- at least one active substance incorporated into the viscoelastic material, that is slowly released from the viscoelastic material when the latter is arranged in contact with the skin.

9. Product according to claim 8, wherein the viscoelastic material is a silicone gel.

10. Product according to claim 9, wherein the silicone gel has mechanical properties similar to the human footpad.

11. Product according to one of claims 8 to 10, for the prevention or care of the consequences of plantar hyperhidrosis, wherein the active substance is chosen from the group comprising fungistats, fungicides, bacteriostats and bactericides.

12. Product according to one of claims 8 to 11, spherical or ovoid in shape and of a diameter adapted to the interdigital space targeted.

13. Product according to one of claims 8 to 11, cylindrical in shape and of a diameter adapted to the interdigital space targeted.

14. Product (1, 1') for applying an active substance to an area of the human body, **characterized in that** it comprises:
- a viscoelastic material spherical or ovoid in shape, having a minimum diameter in the order of 2 mm for an application to the eye or in the order of 4 mm for an application to another area of the human body, and
- at least one active substance incorporated into the viscoelastic material, that is slowly released from the viscoelastic material when the latter is arranged in contact with the skin.

15. Product according to claim 14, wherein the viscoelastic material is a silicone gel.

16. Product according to claim 15, wherein the silicone gel has mechanical properties similar to that of the human tissue in the targeted area of the human body.

17. Product for applying an active substance to an area of the human body, **characterized in that** it comprises:
- a viscoelastic material cylindrical in shape and of a minimum diameter of 4 mm, and
- at least one active substance incorporated into the viscoelastic material, that is slowly released from the viscoelastic material when the latter is arranged in contact with the skin.

18. Product according to claim 17, wherein the viscoelastic material is a silicone gel.

19. Product according to claim 18, wherein the silicone gel has mechanical properties similar to that of the human tissue in the targeted area of the human body.

20. A method for manufacturing a viscoelastic product (1, 1') that is spherical or ovoid in shape and intended for the application of an active substance to an area (2) of the human body, **characterized in that** it comprises the following steps of:
- preparing a mixture comprising at least one compound intended to crosslink in order to form, after cross-linking, a material having viscoelastic properties, and at least one active substance miscible with the compound,
- introducing a determined volume of the mixture into a container containing a fluid not miscible with the mixture, and
- cross-linking the volume of mixture immersed in the fluid.

21. Method according to claim 20, for manufacturing a viscoelastic product as a silicone gel, wherein the mixture comprises at least one silicone oil.

22. Method according to one of claims 20 and 21, wherein the active substance is chosen from the group comprising fungistats, fungicides, bacteriostats and bactericides.

23. Method according to one of claims 20 to 22, wherein the density of the fluid is chosen so that the mixture remains in suspension in the fluid while being immersed, or descends towards the bottom of the container at a speed sufficiently slow to ensure it is cross-linked before reaching the bottom of the container.

24. Method according to one of claims 20 to 23, wherein the fluid is heated to speed up the cross-linking process.

## Patentansprüche

1. Verfahren zur Anwendung einer aktiven Substanz im Bereich des Fußes (2), welches aus ästhetischen, nicht therapeutischen Gründen eingesetzt wird, **dadurch gekennzeichnet, dass** es die Anwendung in mindestens einem Zehenzwischenraum (2) eines viskoelastischen Stoffes (1, 1') umfasst, dessen Form vorgesehen ist, um in den erwähnten Zehenzwischenraum eingesetzt und dort durch Einklemmen zwischen den Zehen gehalten zu werden, wobei der Stoff eine aktive Substanz enthält, welche sich bei Kontakt mit der Haut langsam freisetzt.

2. Verfahren nach Anspruch 1, bei dem der viskoelastische Stoff ein Silikongel ist.

3. Verfahren nach Anspruch 2, bei dem das Silikongel mechanische Eigenschaften ähnlich denen des menschlichen Fußsohlenpolsters aufweist.

4. Verfahren nach einem der Ansprüche 1 bis 3, bei dem die Substanz eine Substanz ist, die darauf abzielt, das Auftreten von unangenehmen Gerüchen im Bereich des Fußes zu beseitigen oder andernfalls zu begrenzen.

5. Verfahren nach einem der Ansprüche 1 bis 4, bei dem der viskoelastische Stoff eine kugel- oder eiförmige Form und einen an den erwähnten Zehenzwischenraum angepassten Durchmesser aufweist.

6. Verfahren nach einem der Ansprüche 1 bis 4, bei dem der viskoelastische Stoff eine zylindrische Form und einen an den erwähnten Zehenzwischenraum angepassten Durchmesser aufweist.

7. Verfahren nach einem der Ansprüche 5 und 6, bei dem der viskoelastische Stoff einen minimalen Durchmesser von ungefähr 4 mm aufweist.

8. Produkt (1, 1') für die Anwendung einer aktiven Substanz an einer Zone des Fußes, **dadurch gekennzeichnet, dass** es Folgendes umfasst:
- einen viskoelastischen Stoff, dessen Form vorgesehen ist, um in einen Zehenzwischenraum (2) eingesetzt und dort durch Einklemmen zwischen den Zehen gehalten zu werden, und
- mindestens eine aktive Substanz, welche dem viskoelastischen Stoff beigemischt ist und sich langsam aus dem viskoelastischen Stoff freisetzt, wenn dieser mit der Haut in Kontakt gebracht wird.

9. Produkt nach Anspruch 8, bei dem der viskoelastische Stoff ein Silikongel ist.

10. Produkt nach Anspruch 9, bei dem das Silikongel mechanische Eigenschaften ähnlich denen des menschlichen Fußsohlenpolsters aufweist.

11. Produkt nach einem der Ansprüche 8 bis 10, für die Vorbeugung oder Behandlung der Folgen der Hyperhidrose der Fußsohlen, bei dem die aktive Substanz aus der Gruppe bestehend aus Fungistatika, Fungiziden, Bakteriostatika und Bakteriziden ausgewählt wird.

12. Produkt nach einem der Ansprüche 8 bis 11, mit kugel- oder eiförmiger Form und einem an den erwähnten Zehenzwischenraum angepassten Durchmesser.

13. Produkt nach einem der Ansprüche 8 bis 11, mit zylindrischer Form und einem an den erwähnten Zehenzwischenraum angepassten Durchmesser.

14. Produkt (1, 1') für die Anwendung einer aktiven Substanz an einer Zone des menschlichen Körpers, **dadurch gekennzeichnet, dass** es Folgendes umfasst:
- einen viskoelastischen Stoff mit kugel- oder eiförmiger Form, der einen minimalen Durchmesser von ungefähr 2 mm für eine Anwendung am Auge oder von ungefähr 4 mm für eine Anwendung in einer anderen Zone des menschlichen Körpers aufweist, und
- mindestens eine aktive Substanz, welche dem viskoelastischen Stoff beigemischt ist und sich langsam aus dem viskoelastischen Stoff freisetzt, wenn dieser mit der Haut in Kontakt gebracht wird.

15. Produkt nach Anspruch 14, bei dem der viskoelastische Stoff ein Silikongel ist.

16. Produkt nach Anspruch 15, bei dem das Silikongel mechanische Eigenschaften ähnlich denen des menschlichen Gewebes in der jeweiligen Zone des menschlichen Körpers aufweist.

17. Produkt für die Anwendung einer aktiven Substanz an einer Zone des menschlichen Körpers, **dadurch gekennzeichnet, dass** es Folgendes umfasst:
- einen viskoelastischen Stoff mit zylindrischer Form und einem minimalen Durchmesser von ungefähr 4 mm, und
- mindestens eine aktive Substanz, welche dem viskoelastischen Stoff beigemischt ist und sich langsam aus dem viskoelastischen Stoff freisetzt, wenn dieser mit der Haut in Kontakt gebracht wird.

18. Produkt nach Anspruch 17, bei dem der viskoelastische Stoff ein Silikongel ist.

19. Produkt nach Anspruch 18, bei dem das Silikongel mechanische Eigenschaften ähnlich denen des menschlichen Gewebes in der jeweiligen Zone des menschlichen Körpers aufweist.

20. Verfahren zur Herstellung eines viskoelastischen Produkts (1, 1') mit kugel- oder eiförmiger Form, welches für die Anwendung einer aktiven Substanz am einer Zone (2) des menschlichen Körpers bestimmt ist, **dadurch gekennzeichnet, dass** es die folgenden Schritte umfasst:
- Vorbereitung eines Gemisches, umfassend mindestens ein Präparat, das dazu bestimmt ist zu vernetzen, um nach der Vernetzung einen Stoff zu bilden, der viskoelastische Eigenschaften besitzt, und mindestens eine aktive, mit dem Präparat mischbare Substanz,
- Einführen einer bestimmten Menge des Gemisches in einen Behälter, welcher ein mit dem Gemisch nicht mischbares Fluid enthält, und
- Vernetzung der Menge des in das Fluid eingetauchten Gemischs.

21. Verfahren nach Anspruch 20, für die Herstellung eines viskoelastischen Produkts aus Silikongel, bei dem das Gemisch mindestens ein Silikonöl umfasst.

22. Verfahren nach einem der Ansprüche 20 und 21, bei dem die aktive Substanz aus der Gruppe bestehend aus Fungistatika, Fungiziden, Bakteriostatika und Bakteriziden ausgewählt wird.

23. Verfahren nach einem der Ansprüche 20 bis 22, bei dem die volumenbezogene Masse des Fluids so ausgewählt wird, dass das Gemisch im Fluid in Suspension und eingetaucht bleibt, oder in Richtung des Bodens des Gefäßes mit einer ausreichend langsamen Geschwindigkeit herabsinkt, damit seine Vernetzung vor dem Erreichen des Bodens des Gefäßes gewährleistet wird.

24. Verfahren nach einem der Ansprüche 20 bis 23, bei dem das Fluid erwärmt wird, um den Vernetzungsprozess zu beschleunigen.
